# EUROPEAN PATENT APPLICATION

(11) **EP 4 310 197 A1**
(43) Date of publication of application: **24.01.2024**
(21) Application number: 22382701.5
(22) Date of filing: 21.07.2022
(51) Int. Cl.: C12Q 1/6886

(54) **METHOD FOR IDENTIFYING LUNG CANCER PATIENTS FOR A COMBINATION TREATMENT OF IMMUNO- AND CHEMOTHERAPY**

(71) Applicant: Fundación para la Investigación Biomédica del Hospital Universitario Puerta de Hierro Majadahonda, 28222 Majadahonda, Madrid (ES); Universidad Autónoma de Madrid, 28049 Madrid (ES); Fundacion GECP, 08027 Barcelona (ES)
(72) Inventor: ROMERO ALFONSO, Atocha, 28222 Majadahonda, Madrid (ES); SERNA BLASCO, Roberto, 28222 Majadahonda, Madrid (ES); PROVENCIO PULLA, Mariano, 28049 Madrid (ES)
(74) Representative: Hoffmann Eitle

(57) **Abstract**

The present invention relates to the field of *in vitro* methods for identifying a subject suitable for treatment with a combination of immunotherapy and chemotherapy by identifying a specific genetic signature in a biological sample from said subject as well as diagnostic devices for performing said method.

## Description

### Technical field

The present invention relates to the field of *in vitro* methods for identifying a subject suitable for treatment with a combination of immuno- and chemotherapy by identifying a specific genetic signature in a biological sample from said subject as well as diagnostic devices for performing said method.

### Background of the invention

Neoadjuvant chemotherapy has been shown to be effective in the treatment of locally advanced NSCLC (1), although neoadjuvant studies over the last 30 years have failed to achieve an impact on overall survival. Recently, studies focusing on the role of immunotherapy alone have reported pathological complete response rates of around 7% (2). However, the combination of immunotherapy plus chemotherapy has proven to be highly effective in these patients, which is leading to a paradigm shift in the treatment of locally advanced NSCLC. The NADIM study (NCT03081689), which evaluates the efficacy of the combination of chemotherapy plus neoadjuvant immunotherapy in stage IIIA patients, has obtained very encouraging results with pathological complete response rates of 63% (3). These results are confirmed by the CheckMate816 clinical trial (4). This has led the FDA to approve this combination in patients with operable stage NSCLC in March 2022. The combination treatment has also been shown to be highly effective in the treatment of advanced disease.

Although immunotherapy has become the standard of care in many cases, to date no robust biomarkers exist for identifying the patients who benefit most from these therapies. Currently used are PD-L1 staining or the tumor mutational load (TMB) for identifying patients who can potentially benefit from immunotherapy. The standard first-line treatment of choice in patients diagnosed with advanced stage NSCLC without molecular target is immunotherapy for tumours with PD-L1 expression≥50% or immunotherapy plus chemotherapy in the remaining patients. Stage IV patients with PD-L1 expression≥50% are candidates for pembrolizumab (5). However, not all PD-L1-positive tumors respond to immunotherapy. On the other hand, high TMB has been associated with better responses to the combination of ipilimumab and nivolumab (6).

WO2020/086724 A1 and WO2019/191676 A1 disclose methods for treating a subject afflicted with a tumor, such as NSCLC, with a combination of an anti-PD-1/PD-L1 antibody and an anti-CTLA-4 antibody and chemotherapy, where the TMB status is determined by sequencing nucleic acids in the tumor and identifying a genomic alteration in the sequenced nucleic acids. WO 2018/183928 A1 discloses a method for identifying a cancer patient, suitable for treatment with immunotherapy, e.g., treatment with an anti-PD-1 antibody or antigen-binding portion thereof, comprising measuring a TMB status of a biological sample of the patient. In these patent applications the TMB of a large set of genes is determined. No specific smaller set of biomarkers is identified that can predict success of treatment.

A combination of chemotherapy plus immunotherapy has been shown to be highly effective in metastatic NSCLC patients and it is frequently used in daily oncology. Nevertheless, classical biomarkers such as PD-L1 or TMB do not serve to select patients who are candidates for chemotherapy-immunotherapy combinations. For example, the KEYNOTE-189 study (NCT02578680) demonstrated that the addition of pembrolizumab to chemotherapy as first-line treatment significantly improved both progression-free survival (PFS) and overall survival (OS) in NSCLC patients with metastatic disease (7), regardless of PDL1 expression mutational burden (8). Similarly, other studies have observed that PD-L1 staining does not predict pathological response rate after neoadjuvant chemotherapy plus sequential immunotherapy, nor is it associated with *downstaging* (9)*.*

As for in the metastatic setting the combination of chemotherapy plus immunotherapy is seen as a new standard of treatment in patients with locally advanced and resectable tumours. However, these treatments have some limitations such as acute and long-term toxicity, as well as having a high economic impact. It is important to recognise that not all patients respond to these treatments and to date no biomarkers have been identified that effectively discriminate those patients who derive long-term benefit from the combination of chemotherapy plus immunotherapy from those who show little benefit from these treatments. In the NADIM study, neither TMB nor PD-L1 were predictors of long-term survival in patients undergoing chemoimmunotherapy (10), again demonstrating that classical biomarkers for immunotherapy are not suitable for immunotherapy plus chemotherapy combinations.

The availability of biomarkers that predict response to treatment is essential for the development of personalized medicine, avoiding ineffective treatments and unnecessary toxicities in non-responder patients, thus allowing efficient management of these drugs, which have a high economic impact. On the other hand, identifying patients at higher risk of disease progression or relapse can help to tailor subsequent treatments based on this risk, in this sense it is possible to adjust the doses or duration of treatments or plan subsequent lines of treatment, prioritizing the course of the disease, thus allowing for rapid and effective management of these patients.

There is, therefore, a need for a method of analysis to identify those patients who achieve long survival as a consequence of these treatments, as well as those who do not benefit from these therapies and who are unnecessarily subjected to treatment-associated toxicity, with little benefit and in whom the administration of other potentially effective treatments is delayed.

### Summary of the invention

The present invention therefore relates in one aspect to an *in vitro* method for identifying a subject suitable for treatment with a combination of immunotherapy and chemotherapy, comprising determining the absence of a somatic mutation in at least two of the genes *EGFR, KMT2C, APC, ERBB3,* and *HNF1A,* and/or the absence of a germline mutation in *PBRM1* in a biological sample from the subject, wherein the subject is afflicted with cancer.

In one embodiment the absence of a somatic mutation in at least two of the genes *EGFR, KMT2C, APC, ERBB3,* and *HNF1A,* and/or the absence of a germline mutation in *PBRM1* in a biological sample from the subject indicates that said subject is more likely to respond to a combination of immunotherapy and chemotherapy than a subject not having said absence.

In one embodiment the method of present invention further comprises determining the absence of a somatic mutation in at least three, at least four, or in all of the genes *EGFR, KMT2C, APC, ERBB3,* and *HNF1A,* and/or the absence of a germline mutation in *PBRM1.*

In a preferred embodiment the germline mutation in *PBRM1* is variant c.1443+60T>G.

In another preferred embodiment the cancer is lung cancer, preferably non-small cell lung cancer (NSCLC).

In yet a further embodiment of the method of present invention the immunotherapy comprises a therapy using PD-1 or PD-L1 antagonists, preferably wherein said PD-1 or PD-L1 antagonists are selected from Pembrolizumab, Nivolumab, Atezolizumab, Ipilimumab, and/or Durvalumab.

In a further embodiment of the method of present invention the chemotherapy comprises the use of a chemotherapeutic agent selected from paclitaxel, carboplatin, docetaxel, oxaliplatin, cisplatin, or any combination thereof.

In one embodiment of the method of present invention the biological sample comprises a tumor tissue biopsy, a liquid biopsy, blood, serum, plasma, genomic DNA, circulating tumor cells, ctDNA, cfDNA, or any combination thereof.

In a further aspect the present invention relates to a diagnostic device/kit for determining the absence of a somatic mutation in at least one of the genes *EGFR, KMT2C, APC, ERBB3* and *HNF1A,* and/or the absence of a germline mutation in *PBRM1* in a biological sample from a subject afflicted with cancer.

In one embodiment of the diagnostic device/kit the absence of a somatic mutation in at least two of the genes *EGFR, KMT2C, APC, ERBB3* and *HNF1A,* and/or the absence of a germline mutation in *PBRM1* in a biological sample from the subject indicates that said subject is more likely to respond to a combination of immunotherapy and chemotherapy than a subject not having said absence.

In a further embodiment of the diagnostic device/kit the cancer is lung cancer, preferably non-small cell lung cancer (NSCLC).

In yet a further embodiment of the diagnostic device/kit the immunotherapy comprises a therapy using PD-1 or PD-L1 antagonists, preferably selected from Pembrolizumab, Nivolumab Atezolizumab, Ipilimumab and/or Durvalumab.

In another embodiment of the diagnostic device/kit of present invention the biological sample comprises a tumor tissue biopsy, a liquid biopsy, blood, serum, plasma, circulating tumor cells, ctDNA, cfDNA, or any combination thereof.

In a further embodiment of the diagnostic device/kit the germline mutation in *PBRM1* is variant c.1443+60T>G.

In a further aspect the present invention relates to the *in vitro* use of the absence of a somatic mutation in at least two of the genes *EGFR, KMT2C, APC, ERBB3,* or *HNF1A,* or the absence of the germline mutation c.1443+60T>G in *PBRM1* as biomarkers in determining whether an anticancer effect is likely to be produced in a cancer by a combination immunotherapy and chemotherapy, in particular immunotherapy with PD-1 or PD-L1 antagonists.

### Brief description of the figures

**Figure 1****:** Kaplan Meier curves for PFS and OS as a function of mutational status in one of the *APC, KMT2C* and *ERBB3* genes.
**Figure 2****:** Kaplan Meier curves for PFS and OS as a function of mutational status in *KMT2C* and *ERBB3* independently.
**Figure 3****:** Population allele frequency of the c.1443+60T>G variant of *PBRM1.*

### Detailed Description

In recent years, medical oncologists have witnessed unprecedented progress in the treatment of many tumours where, despite huge international efforts, therapeutic advances had been very poor, with very few positive clinical trials. Such was the case for the treatment of lung cancer without molecular targets. In this regard, immunotherapy has offered spectacular results and has established itself as the first treatment option in the majority of patients with non-small cell lung cancer (NSCLC) with metastatic disease without molecular targets.

Immune checkpoints are proteins expressed by certain types of immune system cells, such as T cells, and by some tumour cells. These proteins help control immune responses and can prevent T cells from destroying cancer cells. When the action of these proteins is blocked, T cells are better able to kill cancer cells. PD-1/PD-L1 and CTLA-4/B7-1/B7-2 are examples of checkpoint proteins that are present on T cells or tumour cells.

Currently, antibodies targeting immune checkpoints, which block PD-1 or PD-L1, are approved for the treatment of multiple types of advanced (metastatic) cancer and for the broad and genetically defined category of tumours with high microsatellite instability. They are also approved in adjuvant for melanoma and are under investigation in adjuvant and/or neoadjuvant protocols in several tumour types. Anti-PD-L1 therapy is generally well tolerated, with grade 3 to 4 treatment-related adverse events observed in approximately 10% to 20% of patients.

Unlike chemotherapy and molecularly targeted therapy, immune checkpoint blockade immunotherapy results in durable clinical responses through induction, activation and expansion of tumour-specific cytotoxic T cells. However, there are no biomarkers that unequivocally identify long survivors. Biomarkers currently used include immunohistochemical expression of PD-L1 and TMB or mutational load. Neither of these two approaches identifies all responders and not all patients with tumours "positive" for these techniques ultimately turn out to be long survivors, which in these tumour types are defined as patients with more than 2 years of overall survival.

Mutations in the STK11/LKB1/KEAP1 tumour suppressor genes correlate with the presence of KRAS mutations, representing more aggressive subtypes of NSCLC, and have been recognised as primary resistance mutations to PD-1 inhibitors in KRAS-mutated lung adenocarcinoma (11). On the other hand, it is known that EGFR-mutated tumours do not benefit from immunotherapy, as demonstrated by subgroup analysis of the CheckMate 057 (12) and KeyNote studies 010 (13).

On the other hand, therapeutic responses to immunotherapy are difficult to assess using traditional methods based on image-based tumour size measurement (PET or PET-CT), because the immune infiltration of the tumour does not allow for a good delineation of tumour size and therefore it is difficult to measure response based on tumour shrinkage.

In addition, immunotherapy in combination with chemotherapy has proven to be highly effective for first-line treatment of NSCLC with advanced or locally advanced disease in patients in whom immunotherapy alone is not the first treatment option. However, there are currently no biomarkers for this combination. Neither TMB nor PD-L1 expression predicts response to these treatments (6,7,10,14).

The success of these combinations in patients with advanced disease has led to enormous interest in the use of immunotherapy plus chemotherapy combinations in earlier, curable stages of cancer.

Approximately one third of NSCLC patients are diagnosed at stage III. These patients may be offered neoadjuvant chemotherapy in order to shrink the tumour and increase the success rate of surgery with curative intent. However, with current treatments, less than 30% of these patients are alive at three years (15). In this sense, the NADIM clinical trial (NCT03081689), which evaluates the efficacy of the combination of chemotherapy plus immunotherapy in stage IIIA patients, has obtained very encouraging results with pathological complete response rates of 63%, with more than 80% of patients alive at three years, which represents a paradigm shift in the treatment of lung cancer. These results have been supported by data from the randomised phase III phase 3 CheckMate 816 trial, which showed that neoadjuvant nivolumab plus chemotherapy increased the pathological complete response rate compared to the standardised phase III trial.

As described above, neither TMB nor PD-L1 proved to be of prognostic value in the NADIM study (10). Similarly, other studies have observed that PD-L1 staining does not predict the pathological response rate after neoadjuvant treatment with chemotherapy plus sequential immunotherapy, nor is it associated with downstaging (8). This again shows that classical biomarkers for immunotherapy are not suitable for immunotherapy plus chemotherapy combinations.

In order to overcome this problem, the inventors have analysed samples from the above mentioned NADIM trial, as further detailed in Example 1 below, and have identified several somatic mutations that predispose a subject afflicted with cancer, specifically NSCLC, to a worse prognosis. Specifically, the inventors identified five genes *EGFR, KMT2C, APC, ERBB3* and *HNF1A* that significantly predict long-term survival in patients included in the NADIM study. Patients whose tumours carry mutations in any of these genes have significantly lower PFS and OS than those with tumours without these mutations (P LogRank <0.05 for PFS and OS). In addition, a sixth gene could be identified that is suitable for prediction of the suitability for a treatment of immunotherapy plus chemotherapy in NSCLC patients, namely the *PBRM1* gene. Patients carrying the germline variant c.1443+60T>G in the *PBRM1* gene had worse PFS and OS (HR:52.81; 95CI: 4.7-597; P adjusted value by Bonferroni method<0.001 and HR: 15.81; 95%CI 2.6-96.7; P: 0.025 for PFS and OS, respectively.

The authors of the present invention have thus identified a set of genes that are significantly associated with an increased likelihood of disease progression and increased risk of death by analysing a homogeneous cohort of patients treated with chemotherapy plus immunotherapy in neoadjuvant therapy.

The present invention therefore in one aspect relates to an *in vitro* method for identifying a subject suitable for treatment with a combination of immunotherapy and chemotherapy, comprising determining the absence of a somatic mutation in at least two of the genes *EGFR, KMT2C, APC, ERBB3,* and *HNF1A,* and/or the absence of a germline mutation in *PBRM1,* preferably the germline mutation c.1443+60T>G in *PBRM1,* in a biological sample from the subject, wherein the subject is afflicted with cancer.

In one embodiment of present invention the absence of a somatic mutation in at least two of the genes *EGFR, KMT2C, APC, ERBB3,* and *HNF1A,* and/or the absence of a germline mutation in *PBRM1,* preferably the germline mutation c.1443+60T>G in *PBRM1,* in a biological sample from the subject indicates that said subject is more likely to respond to a combination of immunotherapy and chemotherapy than a subject not having said absence.

It is to be understood that the reference group for comparing the response or likelihood of response is made up of subjects not having said absence of a somatic mutation as referred to herein above. For clarity, this means that for example a subject with an absence of a somatic mutation in genes *EGFR* and *KMT2C* in a biological sample from said subject is compared to a subject having a somatic mutation in genes *EGFR* and *KMT2C* in a biological sample from said subject.

As used herein the term "more likely to respond" means that at least 50%, more preferred at least 60%, most preferred at least 70% of subjects will respond to a combination of immunotherapy and chemotherapy. In one embodiment at least 50%, 55%, 60%, 65%, 70%, 75%, 80% of subjects will respond to a combination of immunotherapy and chemotherapy.

In this regard it could be shown by the inventors that patients whose tumours harbour a mutation in at least one of the following genes *EGFR, KMT2C, APC, ERBB3,* and *HNF1A* are at risk of progression HR: 33.6 (95%CI3.61-313.7). Similarly, patients harbouring the c.1443+60T>G have inferior progression-free survival (HR: 52.81; 95%CI: 4.7-597).

In a preferred embodiment of present invention the absence of a somatic mutation in at least three, at least four, or in all of the genes *EGFR, KMT2C, APC, ERBB3,* and *HNF1A,* and/or the absence of a germline mutation in *PBRM1,* preferably the germline mutation c.1443+60T>G in *PBRM1,* in a biological sample from the subject indicates that said subject is more likely to respond to a combination of immunotherapy and chemotherapy than a subject not having said absence.

*KMT2C* is considered to be a tumour suppressor gene that encodes for a nuclear protein with DNA binding domains. It participates in histone methylation and is involved in transcription-related processes. Pathogenic mutations in this gene have been associated with increased TMB and PD-L1 expression and improved OS in patients treated with immunotherapy (21). However, there exists no data on the combination of chemotherapy plus immunotherapy. In myelodysplastic syndromes, pathogenic mutations in this gene enhance the self-renewal capacity of haematopoietic stem cells and confer a selective advantage after chemotherapy (22).

*APC* is a tumour suppressor gene that acts as an antagonist of the Wnt pathway. It is involved in processes such as cell migration and adhesion as well as apoptosis. Pathogenic mutations in this gene via the germline pathway lead to familial adenomatous polyposis, a syndrome that confers increased susceptibility to the development of colorectal polyps and the development of cancer. Mutations in the Wnt pathway are frequent in NSCLC tumours. The Wnt pathway has also been implicated in the immune response to tumours (17,18). However, to date no study has documented that pathogenic mutations in this gene have prognostic value in patients treated with immunotherapy. In this regard, the group led by Dr. Enriqueta Felip et al. found no association between the prognosis of NSCLC patients with advanced disease treated with immunotherapy and APC mutational status (19). Pathogenic mutations in APC have been linked to platinum resistance in vitro studies (20).

*ERBB3* is a member of the epidermal growth factor receptor tyrosine kinase *(EGFR)* family of receptors. This membrane-bound protein has a neurregulin binding domain but no active kinase domain. Therefore, it can bind to this ligand but not transmit the signal to the cell through protein phosphorylation. However, it forms heterodimers with other members of the EGF receptor family that have kinase activity. Heterodimerisation leads to activation of pathways leading to cell proliferation or differentiation. The molecular mechanisms by which *ERBB3* could mediate resistance to these treatments would be similar to those of *EGFR.* No published data associating mutations in this gene with resistance to immunotherapy exists, but in vitro studies show that inactivation of HER3 increases the response to anti-PD-1 blockade in mouse models (23).

*HNF1A* is a transcription factor that is highly expressed in the liver and is involved in the regulation of the expression of several genes. Mutations in this gene have not been associated with immunotherapy response but with resistance to gemcitabine in pancreatic cancer (24).

*PBRM1* encodes a subunit of ATP-dependent chromatin remodelling complexes. The encoded protein has been identified as an integral component of the complexes required for ligand-dependent transcriptional activation by nuclear hormone receptors. Mutations at this locus have been associated with primary clear cell renal cell carcinoma.

In a preferred embodiment of present invention, the absence of a somatic mutation in the below listed genes in a biological sample from a subject indicate that said subject is more likely to respond to a combination of immunotherapy and chemotherapy:

### Biomarkers of present invention

| Absence of somatic mutation in gene(s) | Absence of germline mutation c.1443+60T>G in gene *PBRM1* (yes / no) | |
|---|---|---|
| *EGFR, KMT2C* | yes | no |
| *EGFR, KMT2C, APC* | | |
| *EGFR, KMT2C, ERBB3* | | |
| *EGFR, KMT2C, HNF1A* | | |
| *EGFR, KMT2C, ERBB3, HNF1A* | | |
| *EGFR, APC* | | |
| *EGFR, APC, ERBB3* | | |
| *EGFR, APC, HNF1A* | | |
| *EGFR, APC, ERBB3, HNF1A* | | |
| *EGFR,ERBB3* | | |
| *EGFR, ERBB3, HNF1A* | | |
| *EGFR, HNF1A* | | |
| *KMT2C, APC* | | |
| *KMT2C, APC, ERBB3* | | |
| *KMT2C, APC, ERBB3, HNF1A* | | |
| *KMT2C, ERBB3* | | |
| *KMT2C, ERBB3, HNF1A* | | |
| *APC, ERBB3,* | | |
| *APC, ERBB3, HNF1A* | | |
| *ERBB3, HNF1A* | | |

It is to be understood from this table that any of the above listed genes comprising an absence of a somatic mutation can be combined with the absence of the germline mutation c.1443+60T>G in gene *PBRM1.*

Furthermore, in one embodiment of the present invention a germline mutation in *PBRM,* preferably the germline mutation c.1443+60T>G in *PBRM1,* can be combined with the absence of a somatic mutation in only one of the genes *EGFR, KMT2C, APC, ERBB3* or *HNF1A,* as shown in the below table:

| Absence of somatic mutation in gene | Absence of the germline mutation |
|---|---|
| *EGFR* | c.1443+60T>G in gene *PBRM1* |
| *KMT2C* | |
| *APC* | |
| *ERBB3* | |
| *HNF1A* | |

In a further embodiment of present invention, the above biomarkers can be further combined with biomarkers selected from the genes *BRAF, ALK, ROS1.*

Any of the above recited combinations can thus serve as a biomarker for identifying cancer patients that will benefit from a combination treatment of immune- and chemotherapy.

In a preferred embodiment of present invention, the cancer is lung cancer. In a more preferred embodiment, the cancer is non-small cell lung cancer (NSCLC).

There are seven stages of NSCLC: Occult non-small cell lung cancer, Stage 0 (carcinoma in situ), Stage I, Stage II, Stage IIIA, Stage IIIB, and Stage IV. In one embodiment, the subject is afflicted with a Stage III or IV disease. Patients with Stage III or IV disease have a good performance status benefit from chemotherapy. Many drugs, including platinum agents (e.g., cisplatin, carboplatin, oxilaplatin), taxanes agents (e.g., paclitaxel, albumin-bound paclitaxel, and docetaxel), vinorelbine, vinblastine, etoposide, pemetrexed and gemcitabine are useful for the treatment of Stage III and IV NSCLC.

In one preferred embodiment the chemotherapy thus comprises or consists of the use of a chemotherapeutic agent selected from paclitaxel, carboplatin, docetaxel, oxaliplatin, cisplatin, or any combination thereof.

In one embodiment of the method of present invention, the immunotherapy comprises or consists of a therapy using PD-1 or PD-L1 antagonists.

Anti-PD-1 antibodies that are known in the art can be used in the presently described method. Various human monoclonal antibodies that bind specifically to PD-1 with high affinity have been disclosed in U.S. Patent No. 8,008,449. Anti- PD-1 human antibodies disclosed in U.S. Patent No. 8,008,449 have been demonstrated to exhibit one or more of the following characteristics: (a) bind to human PD-1 with a KD of 1 x 10"7 M or less, as determined by surface plasmon resonance using a Biacore biosensor system; (b) do not substantially bind to human CD28, CTLA-4 or ICOS; (c) increase T-cell proliferation in a Mixed Lymphocyte Reaction (MLR) assay; (d) increase interferon-γ production in an MLR assay; (e) increase IL-2 secretion in an MLR assay; (f) bind to human PD-1 and cynomolgus monkey PD-1; (g) inhibit the binding of PD-L1 and/or PD-L2 to PD-1; (h) stimulate antigen-specific memory responses; (i) stimulate antibody responses; and (j) inhibit tumor cell growth in vivo. Anti-PD-1 antibodies usable in the present disclosure include monoclonal antibodies that bind specifically to human PD-1 and exhibit at least one, in some embodiments, at least five, of the preceding characteristics.

Other anti-PD-1 monoclonal antibodies have been described in, for example, U.S.Patent Nos. 6,808,710, 7,488,802, 8,168,757 and 8,354,509, US Publication No. 2016/0272708, and PCT Publication Nos. WO 2012/145493, WO 2008/156712, WO 2015/112900, WO 2012/145493, WO 2015/112800, WO 2014/206107, WO 2015/35606, WO 2015/085847, WO 2014/179664, WO 2017/020291, WO 2017/020858, WO 2016/197367, WO 2017/024515, WO 2017/025051, WO 2017/123557, WO 2016/106159, WO 2014/194302, WO 2017/040790, WO 2017/133540, WO 2017/132827, WO 2017/024465, WO 2017/025016, WO 2017/106061, WO 2017/19846, WO 2017/024465, WO 2017/025016, WO 2017/132825, and WO 2017/133540 each of which is incorporated by reference in its entirety.

In a preferred embodiment of the invention the PD-1 or PD-L1 antagonists are selected from Pembrolizumab, Nivolumab, Atezolizumab, Ipilimumab and/or Durvalumab. Most preferred is Nivolumab.

Pembrolizumab (Merck; also known as KEYTRUDA^{®}, lambrolizumab, and MK-3475; see WO2008/156712) is a humanized monoclonal IgG4 (S228P) antibody directed against human cell surface receptor PD-1.

Nivolumab (formerly designated 5C4, BMS-936558, MDX-1106, or ONO-4538) is a fully human IgG4 (S228P) PD-1 immune checkpoint inhibitor antibody that selectively prevents interaction with PD-1 ligands (PD-L1 and PD-L2), thereby blocking the down- regulation of antitumor T-cell functions. Nivolumab has shown activity in a variety of advanced solid tumors, including non-small cell lung cancer (NSCLC).

Atezolizumab (sold under Tecentriq^{®}) is a fully humanized, engineered monoclonal antibody of IgG1 isotype against against PD-L1 used to treat various cancer types, among them NSCLC.

Ipilimumab (sold under Yervoy^{®}) is a monoclonal antibody targeting CTLA-4, a protein receptor that downregulates the immune system.

Durvalumab (sold under Imfinzi^{®}) is an FDA-approved immunotherapy for cancer, developed by Medimmune/AstraZeneca and is a human immunoglobulin G1 kappa (IgG1κ) monoclonal antibody that blocks the interaction of PD-L1 with PD-1.

In one embodiment of present invention the biological sample comprises a tumor tissue biopsy, a liquid biopsy, blood, serum, plasma, genomic DNA, circulating tumor cells, ctDNA, cfDNA, or any combination thereof.

As described further in Example 1, the inventors have studied the somatic mutational profile in the *EGFR, KMT2C, APC, ERBB3* and *HNF1A* genes, as well as the germline variants c.1443+60T>G, in the *PBRM1* gene to predict long-term PFS and OS in patients with NSCLC undergoing immunotherapy (nivolumab) plus platinum-based chemotherapy (carboplatin, oxaliplatin or cisplatin), such as the combination of a platinum and a taxane (paclitaxel or docetaxel). This requires obtaining a tumour biopsy, with at least 10% of cells in the tumour.

The DNA was isolated and the mutational profile in the *EGFR, KMT2C, APC, ERBB3* and *HNF1A* genes studied. Furthermore, a blood sample was isolated to study the germline variants c.1443+60T>G in the *PBRM1* gene Based on this study, patients could be categorised as high or low risk.

The inventors could show that patients whose tumours carry mutations in any of the *EGFR, KMT2C, APC, ERBB3* and/or *HNF1A* genes have significantly lower PFS and OS than those with tumours that do not (P LogRank <0.05 for PFS and OS). Table 1 shows the Hazard Ratio (HR) for PFS as well as the Harrell's C-index statistic, which shows the ability of each of the genes to discriminate between responders and non-responders in terms of PFS. Table 2 shows the HR and C-index for OS for each of the signature and combined genes.

**Table 1: Hazard Ratio (HR) and Harrell's C-index statistic for PFS**

| | **Progression-free survival (PFS)** | | | | | | |
|---|---|---|---|---|---|---|---|
| | **Univariable** | | **Multivariable** | | **C-Index** | | **LogRank** |
| **Gene** | HR (95% Cl) | P value | HR (95% Cl) | P value | Coef (95% Cl) | P-value | P-value |
| ***APC*** | 4.61(0.95-22.34) | 0.058 | 5.93 (1.14-30.75) | 0.034 | 0.66 (0.50-0.82) | <0.001 | 0.037 |
| ***KMT2C*** | - | - | - | - | 0.59 (0.42-0.76) | <0.001 | <0.001 |
| ***EGFR*** | 12.33(2.41-62.8) | 0.003 | 17.41 (3.1-98.46) | 0.001 | 0.73 (0.58-0.89) | <0.001 | <0.001 |
| ***ERBB3*** | 2.88 (0.59-14.0) | 0.189 | 3.72 (0.71-19.38) | 0.119 | 0.67 (0.48-0.85) | <0.001 | 0.169 |
| ***HNF1A*** | 3.28 (0.41-26.3) | 0.264 | 3.34 (0.40-27.78) | 0.264 | 0.58 (0.40-0.76) | <0.001 | 0.237 |
| ***Combined_5*** | 12.7(2.60-62.07) | 0.002 | 33.6(3.61-313.7) | 0.002 | 0.84 (0.72-0.96) | <0.001 | <0.001 |
| ***Combined_3*** | 7.08(1.88-26.62) | 0.004 | 10.2 (2.32-44.68) | 0.002 | 0.75 (0.59-0.92) | <0.001 | 0.001 |
| ***Com_APC_KMT2C*** | 7.70(1.90-31.16) | 0.004 | 8.84(2.07-67.68) | 0.003 | 0.67 (0.50-0.85) | <0.001 | 0.001 |
| ***Com_APC_ERBB3*** | 4.60(1.23-47.27) | 0.024 | 7.01(1.56-31.54) | 0.011 | 0.73 (0.58-0.87) | <0.001 | 0.013 |
| ***Com_KMT2C_ERBB3*** | 4.93(1.21-20.03) | 0.026 | 5.94(1.38-25.62) | 0.017 | 0.67 (0.49-0.86) | <0.001 | 0.014 |

**Table 2: Hazard Ratio (HR) and Harrell's C-index statistic for OS**

| | **O** S | | | | | | |
|---|---|---|---|---|---|---|---|
| | **Univariable** | | **Multivariable** | | **C-lndex** | | **LogRank** |
| **Gene** | **HR (95% Cl)** | **P value** | **HR (95% Cl)** | **P value** | **Coef (95% Cl)** | **P-value** | **P-value** |
| ***APC*** | - | - | - | - | 0.70 (0.48-0.93) | <0.001 | 0.577 |
| ***KMT2C*** | 32.5(2.03-519.6) | 0.014 | 9.6 (0.37-246.31) | 0.172 | 0.69 (0.45-0.92) | <0.001 | <0.001 |
| ***EGFR*** | 2.67 (0.30-24.01) | 0.38 | 4.89 (0.44-54.14) | 0.196 | 0.76 (0.53-0.99) | <0.001 | 0.361 |
| ***ERBB3*** | 5.41 (0.90-32.51) | 0.065 | 14.7(1.33-163.1) | 0.028 | 0.85 (0.69-1.02) | <0.001 | 0.038 |
| ***HNF1A*** | 7.11 (0.72-69.90) | 0.093 | 4.27 (0.35-51.64) | 0.253 | 0.68 (0.44-0.92) | <0.001 | 0.05 |
| **Combined_5** | 4.02 (0.67-24.19) | 0.128 | 6.5 (0.89-47.65) | 0.066 | 0.81 (0.63-0.99) | <0.001 | 0.01 |
| **Combined_3** | 6.91 (1.14-41.74) | 0.035 | 9.45 (1.33-66.89) | 0.025 | 0.84 (0.67-1.01) | <0.001 | 0.014 |
| ***Com_APC_KMT2C*** | 3.33 (0.37-30.21) | 0.284 | 2.69 (0.27-27.08) | 0.4 | 0.67 (0.42-0.92) | <0.001 | 0.256 |
| ***Com_APC_ERBB3*** | 3.30 (0.55-19.82) | 0.192 | 8.90 (0.80-98.46) | 0.075 | 0.84 (0.66-1.01) | <0.001 | 0.167 |
| ***Com_KMT2C_ERBB3*** | 11.06(1.83-67.02) | 0.009 | 13.26(1.9-92.57) | 0.009 | 0.86 (0.70-1.02) | <0.001 | 0.001 |

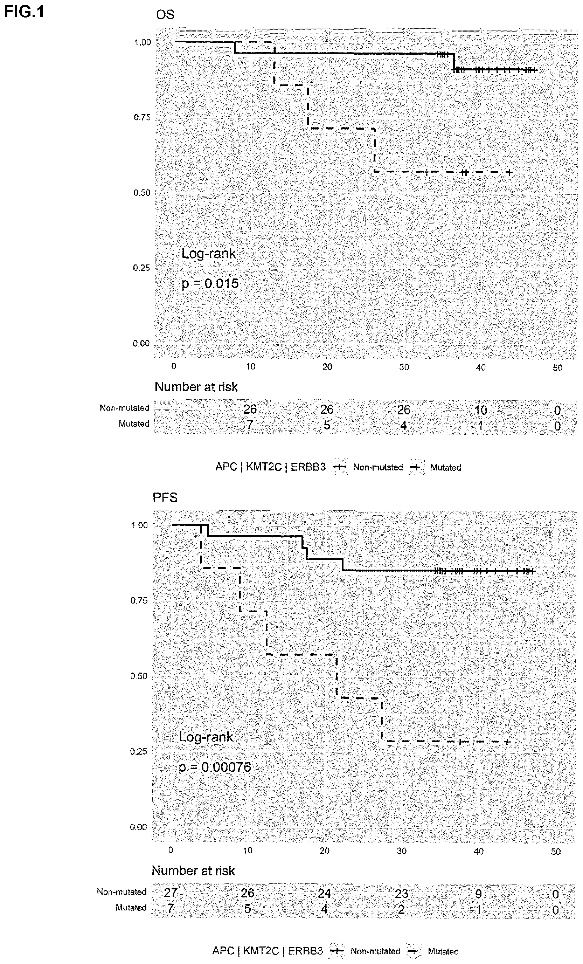

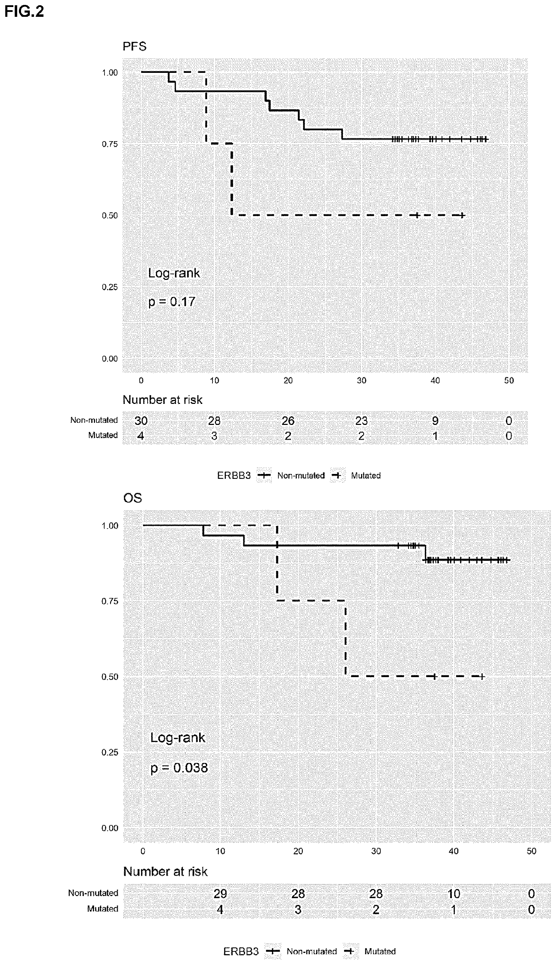

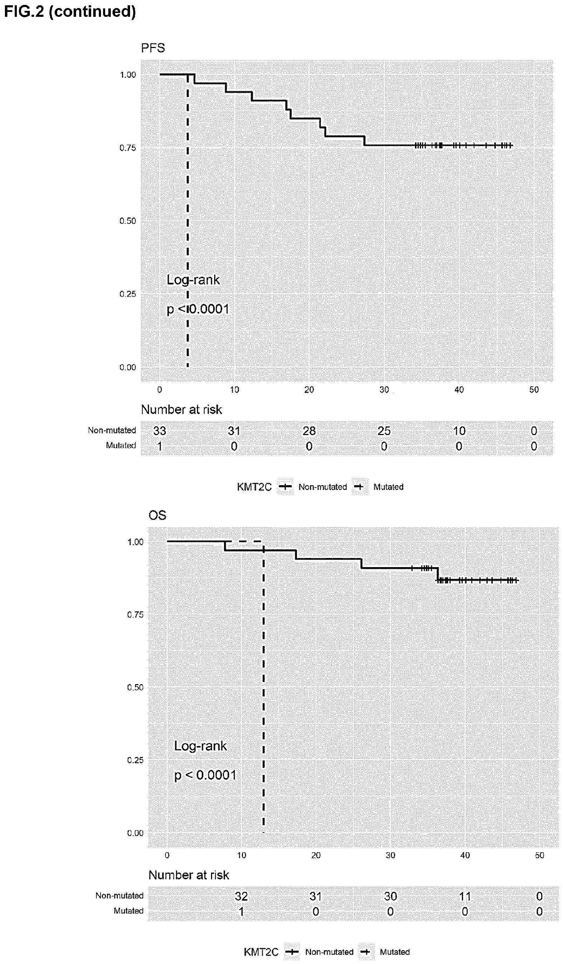

Tables 1 and 2 show that the combination of the *APC, KMT2C* and *ERBB3* genes has the highest predictive capacity in terms of OS (Figure 1). Of these, the combination of the *KMT2C* and *ERBB3* genes appears to be the combination with the highest discriminatory capacity (Figure 2).

Using CRISPR-Cas9-based technology Pan et al (25) identified that inactivation of several genes including *PBRM1* sensitised mouse B16F10 melanoma cells to cytotoxic T-lymphocyte activity. In addition, tumours resistant to immunotherapy treatment responded when this gene was inactivated. In addition, they observed that PBRM1-deficient murine melanomas were more strongly infiltrated by cytotoxic T cells.

To identify genomic alterations in clear cell renal cell carcinoma that correlated with response to anti-PD-1 monotherapy Miao et al (26) performed whole exome sequencing of 35 patients and found that clinical benefit was associated with loss-of-function mutations in the *PBRM1* gene (p=0.012). Miao et al confirmed this finding in an independent validation cohort of 63 patients with clear cell renal cell carcinoma treated with PD-1 or PD-L1 blockade therapy alone or in combination with anti-CTLA4 therapies (p = 0.0071).

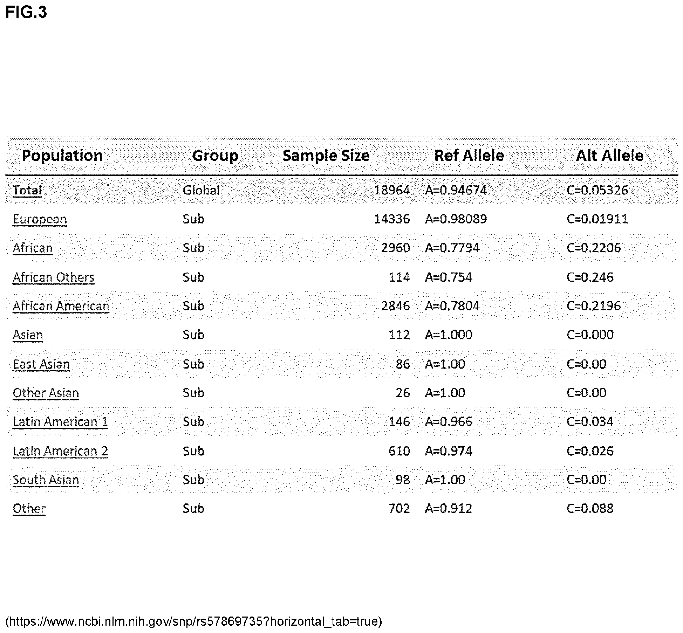

The c.1443+60T>G variant is an intronic variant although it is possible that this variant affects alternative splicing. This is a polymorphic variant with a population allele frequency of around 5%, being more frequent in the African population (Fig. 3).

In a preferred embodiment the present invention refers to a method for discriminating between those patients, diagnosed with NSCLC cancer, with a high probability of obtaining a long-term benefit from the combination of immunotherapy plus chemotherapy, from those who do not obtain a benefit in terms of survival (PFS and OS), by analysing the somatic mutational profile in the *EGFR, KMT2C, APC, ERBB3* and *HNF1A* genes and by studying the germline variants c.1443 +60T>G in the *PBRM1* gene. Preferably, this method allows prediction of clinical benefit (in terms of PFS and OS) to the combination of nivolumab plus carboplatin plus paclitaxel.

During the development of the NADIM clinical study (NCT03081689), whose objective was to evaluate the efficacy of the combination of immunotherapy (specifically the drug nivolumab) plus neoadjuvant chemotherapy (platinum-based) in patients with locally advanced NSCLC, paraffin-embedded samples were collected from lung biopsies prior to the start of neoadjuvant treatment. Genomic DNA was isolated from these samples and analysed by massive sequencing using the Oncomine Tumor Mutation Load Assay panel. After a detailed filtering in which low quality reads, polymorphisms and silent mutations were discarded, a matrix of 754 mutations distributed in 265 genes was obtained. Subsequently, statistical analysis was performed and associations between genes/mutations and treatment response (pathological response and imaging response according to RECIST v1.1 imaging criteria) and survival (PFS and OS) were sought. This analysis provided a list of candidate genes that was finally narrowed down to the following: *EGFR, KMT2C, APC, ERBB3, HNF1A.* Thus, patients whose tumours had mutations in any of these genes had significantly lower PFS and OS than patients whose tumours did not have mutations in any of these genes (P LogRank<0.05 for PFS and OS). On the other hand, a matrix with 31747 potentially germline mutations was obtained. As in the previous case, the inventors searched for associations with survival and found that the c.1443+60T>G variant in the *PBRM1* gene was associated with a worse prognosis (HR:52.81; 95CI: 4.67-597; PBonferroni<0.001 and HR:15.81; 95CI: 2.58-96.74; PBonferroni: 0.025, for SLP and SG, respectively). This variant was found at allele frequencies close to 50% and its germline origin could be validated by sequencing PBMCs of those patients in whom the variant had been identified in the tumour.

The methods as described herein above can be performed by using a diagnostic device or kit as commonly used. The present invention therefore relates in one further aspect to such diagnostic devices or kits applying the method of present invention.

In a preferred embodiment the invention relates to a diagnostic device or kit for determining the absence of a somatic mutation in at least one of the genes *EGFR, KMT2C, APC, ERBB3* and *HNF1A,* and/or the absence of the germline mutation c.1443+60T>G in *PBRM1* in a biological sample from a subject afflicted with cancer.

In one further embodiment of the diagnostic device/kit the absence of a somatic mutation in at least two of the genes *EGFR, KMT2C, APC, ERBB3* and *HNF1A,* and/or the absence of a germline mutation in *PBRM1,* preferably the germline mutation c.1443+60T>G in *PBRM1,* in a biological sample from the subject indicates that said subject is more likely to respond to a combination of immunotherapy and chemotherapy than a subject not having said absence.

It is to be noted that all features as described herein above for the method of present invention are applicable to the diagnostic device or kit.

In a further aspect the present invention relates to the *in vitro* use of the absence of a somatic mutation in at least two of the genes *EGFR, KMT2C, APC, ERBB3* and *HNF1A,* and/or the absence of a germline mutation in *PBRM1,* preferably the germline mutation c.1443+60T>G in *PBRM1,* as biomarkers in determining whether an anticancer effect is likely to be produced in a cancer by a combination immunotherapy and chemotherapy, in particular immunotherapy with PD-1 or PD-L1 antagonists.

Throughout the description and claims the word "comprise" and its variants are not intended to exclude other technical characteristics, additives, components or steps. For those skilled in the art, other objects, advantages and characteristics of the invention will become apparent in part from the description and partly from the practice of the invention. The following examples and figures are provided by way of illustration and are not intended to limit the scope of the present invention.

### EXAMPLES

### Example 1: Identification of Biomarkers from samples of NADIM trial

For the development of the present signature, paraffin-embedded samples of tumour biopsies obtained prior to treatment from patients included in the NADIM study were analysed. Specifically, 34 samples were analysed from the total of 46 patients included in the study. This was a phase 2, open-label, multicentre, single-arm, multicentre trial involving patients with histologically or cytologically documented stage IIIA NSCLC with an Eastern Cooperative Oncology Group performance status of 0 or 1 and who were deemed locally and surgically resectable by a multidisciplinary medical team. Patients were treated with neoadjuvant intravenous paclitaxel (200 mg/m2) and carboplatin (area under the curve 6; 6 mg/mL per minute) plus nivolumab (360 mg) on day 1 of each 21-day cycle for three cycles before surgical resection, followed by adjuvant intravenous nivolumab monotherapy for 1 year (240 mg, every 2 weeks, for 4 months, followed by 480 mg, every 4 weeks, for 8 months).

For each paraffin-embedded sample, DNA was extracted from 3 paraffin sections at least 10 µm thick using the truXTRAC^{®} FFPE total Nucleic Acid kit (Covaris), according to the manufacturer's recommendations. The extracted DNA was quantified using the Qubit^{®} dsDNA HS kit on the Qubit^{®} 2.0 fluorometer (Thermo Fisher Scientific). To remove deamidated bases, prior to amplification of target genes, 20 ng of DNA were treated with thermolabile uracil-DNA glycosylase (UDG), following the manufacturer's recommendations. Library preparation was performed on an Ion Chef ^{™} system (Thermo Fisher Scientific), using 20 ng of DNA and using the Oncomine Tumor Mutation Load Assay kit (Thermo Fisher Scientific). This panel covers 1.7 megabases (Mb) of 409 genes related to cancer development. The indexed libraries were pooled and adjusted to a final concentration of 50 pM and loaded eight at a time onto Ion 540 ^{™} chips, using the Ion Chef ^{™} system. Finally, the Ion 540^{™} chips were sequenced on an Ion S5^{™} sequencer (Thermo Fisher, Palo Alto, CA, USA). Reads were aligned against the hg19 genome using Torrent Suite 5.12 and the BAM files were transferred to Ion Reporter 5.12 for the extraction of vcfs files. Variant filtering was performed using an internal pipeline (see Figure 2). Germline variants were filtered using a filter chain so that variants present at 1000 Genome Project, NHLBI GO Exome Sequencing Project (ESP) and ExAC allele frequencies above 1 % were discarded. Finally, it was confirmed that the relevant mutations were not germline or due to clonal haematopoiesis by Sanger sequencing of PBMCs. Similarly, the germline origin of the variant c.1443+60T>G in the *PBRM1* gene was confirmed by Sanger sequencing of PBMCs.

The median follow-up time was estimated by the inverse Kaplan-Meier (KM) method. Estimation of median follow-up and the ratio of expected variance to actual variance at 36 and 42 months were used to estimate the maturity of the data.

OS was defined as the time from initiation of neoadjuvant treatment to death from any cause. PFS was defined as the time between initiation of neoadjuvant therapy and disease progression, as assessed by radiological response according to RECIST v1.1 criteria, or death from any cause. Patients who were alive or without the event at the end of follow-up were censored at the time of last contact.

Cox proportional hazards models were used to determine the association of each of the variants/genes with survival data. The models were adjusted for the variable surgery (performed or not performed), as this is the major determinant of survival in these patients. For biomarker analysis, patients who died from COVID19 were excluded. Discrimination ability for each model was assessed using Harrell's concordance index (c-index). The c-index can take values from 0 to 1, with higher values indicating better discrimination. A value of 0.5 does not correspond to better discrimination than chance. Values of p <0.05 were considered statistically significant.

### References

1) Rosell R, et al. A randomized trial comparing preoperative chemotherapy plus surgery with surgery alone in patients with non-small-cell lung cancer. N Engl J Med. 1994 Jan 20;330(3):153-8
2) Cascone T, et al. Neoadjuvant nivolumab or nivolumab plus ipilimumab in operable non-small cell lung cancer: the phase 2 randomized NEOSTAR trial. Nat Med. 2021 Mar;27(3):504-514
3) Provencio M, et al. Neoadjuvant chemotherapy and nivolumab in resectable non-small-cell lung cancer (NADIM): an open-label, multicentre, single-arm, phase 2 trial. Lancet Oncol. 2020 Nov;21(11):1413-1422.
4) Forde, P. M. et al. Nivolumab (NIVO) + platinum-doublet chemotherapy (chemo) vs chemo as neoadjuvant treatment (tx) for resectable (IB-IIIA) non-small cell lung cancer (NSCLC) in the phase 3 CheckMate 816 trial. AACR Annual Meeting. Virtual. Abstract CT003 (2021)
5) Reck M, et al. Pembrolizumab versus Chemotherapy for PD-L1-Positive Non-Small-Cell Lung Cancer. N Engl J Med. 2016 Nov 10;375(19):1823-1833.
6) Hellmann MD, et al. Nivolumab plus Ipilimumab in Lung Cancer with a High Tumor Mutational Burden. N Engl J Med. 2018 May 31;378(22):2093-2104.
7) Gandhi L, et al. Pembrolizumab plus Chemotherapy in Metastatic Non-Small-Cell Lung Cancer. N Engl J Med. 2018 May 31;378(22):2078-2092
8) Garassino MC, et al. Abstract OA04.06. Presented at: International Association for the Study of Lung Cancer World Conference on Lung Cancer; Sept. 7-10, 2019; Barcelona.
9) Rothschild SI et al. SAKK 16/14: Durvalumab in Addition to Neoadjuvant Chemotherapy in Patients With Stage IIIA(N2) Non-Small-Cell Lung Cancer-A Multicenter Single-Arm Phase II Trial. J Clin Oncol. 2021 Sep 10;39(26):2872-2880
10) Romero A et al. OA20.02 Pre-Treatment Levels of ctDNA for Long-term Survival Prediction in Stage IIIA NSCLC Treated With Neoadjuvant Chemo-Immunotherapy - Nadim Study. IASLC 2021 World Conference on Lung Cancer.
11) Skoulidis F Et al. Co-occurring genomic alterations define major subsets of KRAS-mutant lung adenocarcinoma with distinct biology, immune profiles, and therapeutic vulnerabilities. Cancer Discv. 2015;5:860-877
12) Borghaei H, Paz-Ares L, Horn L, Spigel DR, Steins M, Ready NE, Chow LQ, Vokes EE, Felip E, Holgado E, Barlesi F, Kohlhaufl M, Arrieta O, Burgio MA, Fayette J, Lena H, Poddubskaya E, Gerber DE, Gettinger SN, Rudin CM, Rizvi N, Crino L, Blumenschein GR Jr, Antonia SJ, Dorange C, Harbison CT, Graf Finckenstein F, Brahmer JR. Nivolumab versus docetaxel in advanced nonsquamous non-small-cell lung cancer. N Engl J Med. 2015;373:1627-1639
13) Gandhi L, Rodriguez-Abreu D, Gadgeel S, Esteban E, Felip E, De Angelis F, Domine M, Clingan P, Hochmair MJ, Powell SF, Cheng SY, Bischoff HG, Peled N, Grossi F, Jennens RR, Reck M, Hui R, Garon EB, Boyer M, Rubio-Viqueira B, Novello S, Kurata T, Gray JE, Vida J, Wei Z, Yang J, Raftopoulos H, Pietanza MC, Garassino MC. Pembrolizumab plus chemotherapy in metastatic non-small-cell lung cancer. N Engl J Med. 2018;378:2078-2092
14) Paz-Ares L, Ciuleanu TE, Cobo M, et al. First-line nivolumab plus ipilimumab combined with two cycles of chemotherapy in patients with non-small-cell lung cancer (CheckMate 9LA): an international, randomised, open-label, phase 3 trial. Lancet Oncol 2021; 22: 198-211.
15) Provencio M et al. OA20.01 Long Term Survival in Operable Stage liia Nsclc Patients Treated With Neoadjuvant Nivolumab Plus Chemotherapy - Nadim Study. IASLC 2021 World Conference on Lung Cancer.
16) Forde PM, Spicer J, Lu S, et al. Neoadjuvant Nivolumab plus Chemotherapy in Resectable Lung Cancer. N Engl J Med. 2022 Apr 11. doi: 10.1056/NEJMoa2202170.
17) Ganesh S, et al. RNAi-mediated beta-catenin inhibition to promote T-cell infiltration and antitumor activity in combination with immune checkpoint blockade. J. Clin. Oncol. 2017; 35
18) Luke JJ, et al. Correlation of WNT/beta-catenin pathway activation with immune exclusion across most human cancers. J. Clin. Oncol. 2016; 34:
19) Ros Montana FJ et al. 1989P - WNT pathway mutations (APC/CTNNB1) and immune checkpoint inhibitors (ICI) response in metastatic non-small cell lung cancer (NSCLC) patients. Abstract Book of the 44th ESMO Congress (ESMO 2019) 27 September - 1 October 2019, Barcelona, Spain
20) VanKlompenberg MK. APC selectively mediates response to chemotherapeutic agents in breast cancer. BMC Cancer. 2015 Jun 7;15:457.
21) Chunling Liu et al. Association of KMT2C mutations with favorable outcomes with immune checkpoint inhibitors across multiple tumor types. Meeting Abstract | 2021 ASCO Annual Meeting I. DOI: 10.1200/JCO.2021.39.15_suppl.2600 Journal of Clinical Oncology 39, no. 15_suppl (May 20, 2021) 2600-2600
22) Chen R et al. KMT2C mutations enhance HSC self-renewal capacity and convey a selective advantage after chemotherapy. Cell Rep. 2021 Feb 16;34(7):108751.
23) Wang Z et al. Disruption of the HER3-PI3K-mTOR oncogenic signaling axis and PD-1 blockade as a multimodal precision immunotherapy in head and neck cancer. Nat Commun. 2021 Apr22;12(1):2383
24) Lu Y et al. HNF1A inhibition induces the resistance of pancreatic cancer cells to gemcitabine by targeting ABCB1. EBioMedicine. 2019 Jun; 44: 403-418
25) Pan D, et al. A major chromatin regulator determines resistance of tumor cells to T cell-mediated killing. Science 2018 Feb 16;359(6377):770-775
26) Miao D et al. Genomic correlates of response to immune checkpoint therapies in clear cell renal cell carcinoma. Science. 2018 Feb 16;359(6377):801-806.
27) Nishimura CD et al. c-MPL provides tumor-targeted T-cell receptor-transgenic T cells with costimulation and cytokine signals. Blood. 2017 Dec 21;130(25):2739-2749.

## Claims

1. An *in vitro* method for identifying a subject suitable for treatment with a combination of immunotherapy and chemotherapy, comprising determining the absence of a somatic mutation in at least two of the genes *EGFR, KMT2C, APC, ERBB3,* and *HNF1A,* and/or the absence of a germline mutation in *PBRM1* in a biological sample from the subject, wherein the subject is afflicted with cancer.

2. The method of claim 1, wherein the absence of a somatic mutation in at least two of the genes *EGFR, KMT2C, APC, ERBB3,* and *HNF1A,* and/or the absence of a germline mutation in *PBRM1* in a biological sample from the subject indicates that said subject is more likely to respond to a treatment with a combination of immunotherapy and chemotherapy than a subject not having said absence.

3. The method of any one of the preceding claims, comprising determining the absence of a somatic mutation in at least three, at least four, or in all of the genes *EGFR, KMT2C, APC, ERBB3,* and *HNF1A,* and/or the absence of a germline mutation in *PBRM1.*

4. The method of any one of the preceding claims, wherein the germline mutation in *PBRM1* is variant c.1443+60T>G.

5. The method of any one of the preceding claims, wherein the cancer comprises lung cancer, preferably non-small cell lung cancer (NSCLC).

6. The method of any one of the preceding claims, wherein the immunotherapy comprises a therapy using PD-1 or PD-L1 antagonists, preferably wherein said PD-1 or PD-L1 antagonists are selected from Pembrolizumab, Nivolumab and/or Atezolizumab, Ipilimumab, Durvalumab.

7. The method of any one of the preceding claims, wherein the chemotherapy comprises the use of a chemotherapeutic agent selected from paclitaxel, carboplatin, docetaxel, oxaliplatin, cisplatin, or any combination thereof.

8. The method of any one of the preceding claims, wherein the biological sample comprises a tumor tissue biopsy, a liquid biopsy, blood, serum, plasma, genomic DNA, circulating tumor cells, ctDNA, cfDNA, or any combination thereof.

9. A diagnostic device/kit for determining the absence of a somatic mutation in at least one of the genes *EGFR, KMT2C, APC, ERBB3* and *HNF1A,* and/or the absence of a germline mutation in *PBRM1* in a biological sample from a subject afflicted with cancer.

10. The diagnostic device of claim 9, wherein the absence of a somatic mutation in at least two of the genes *EGFR, KMT2C, APC, ERBB3* and *HNF1A,* and/or the absence of a germline mutation in *PBRM1* in a biological sample from the subject indicates that said subject is more likely to respond to a combination of immunotherapy and chemotherapy than a subject not having said absence.

11. The diagnostic device of any one of claims 9 or 10, wherein the cancer is lung cancer, preferably non-small cell lung cancer (NSCLC).

12. The diagnostic device of any one of claims 9 to 11, wherein the immunotherapy comprises a therapy using PD-1 or PD-L1 antagonists, preferably selected from Pembrolizumab, Nivolumab and/or Atezolizumab, Ipilimumab, Durvalumab.

13. The diagnostic device of any one of claims 9 to 12, wherein the biological sample comprises a tumor tissue biopsy, a liquid biopsy, blood, serum, plasma, circulating tumor cells, ctDNA, cfDNA, or any combination thereof.

14. The diagnostic device of any one of claims 9 to 13, wherein the germline mutation in *PBRM1* is variant c.1443+60T>G.

15. *In vitro* use of the absence of a somatic mutation in at least two of the genes *EGFR, KMT2C, APC, ERBB3* and *HNF1A,* or the absence of a germline mutation in *PBRM1,* preferably the germline mutation c.1443+60T>G in *PBRM1,* as biomarkers in determining whether an anticancer effect is likely to be produced in a cancer by a combination of immunotherapy and chemotherapy, in particular immunotherapy with PD-1 or PD-L1 antagonists.
